Europäisches Patentamt

European Patent Office

Office européen des brevets

(11) Veröffentlichungsnummer: **0 196 037**
**A2**

(12) **EUROPÄISCHE PATENTANMELDUNG**

(21) Anmeldenummer: 86103968.3

(22) Anmeldetag: 22.03.86

(51) Int. Cl.⁴: **C07C 29/04** , C07C 31/12 , C07C 41/09 , C07C 43/04

(30) Priorität: 29.03.85 DE 3511399

(43) Veröffentlichungstag der Anmeldung:
01.10.86 Patentblatt 86/40

(84) Benannte Vertragsstaaten:
AT BE DE FR GB IT NL

(71) Anmelder: BASF Aktiengesellschaft
Carl-Bosch-Strasse 38
D-6700 Ludwigshafen(DE)

(72) Erfinder: Volkamer, Klaus, Dr.
Heidelberger Ring 21
D-6710 Frankenthal(DE)
Erfinder: Lindner, Alfred, Dr.
Ringstrasse 30
D-6712 Bobenheim-Roxheim(DE)
Erfinder: Schwartz, Erich, Dr.
Mohnstrasse 37
D-6700 Ludwigshafen(DE)

(54) Verfahren zur Herstellung von tert.-Butanol und gegebenenfalls Methyl tert.-butylether und/oder Ethyl-tert.-butylether.

(57) Die vorliegende Erfindung betrifft ein Verfahren zur Herstellung von tert.-Butanol durch Umsetzung von Isobuten enthaltenden $C_4$-Kohlenwasserstoffgemischen mit Wasser in Gegenwart von sauren Ionenaustauschern und Abtrennung des gebildeten tert.-Butanols aus dem Reaktionsgemisch, bei dem man einen Teil des bei der Hydratisierung gebildeten tert.-Butanols in die Hydratisierung zurückführt.

EP 0 196 037 A2

Verfahren zur Herstellung von tert.-Butanol und gegebenenfalls Methyl-tert.-butylether und/order Ethyl-tert.-butylether

Die vorliegende Erfindung betrifft ein Verfahren zur Herstellung von tert.-Butanol und gegebenenfalls Methyl-tert.-butylether und/oder Ethyl-tert.-butylether durch Umsetzung von Isobuten enthaltenden $C_4$-Kohlenwasserstoffgemischen mit Wasser und gegebenenfalls Methanol und/oder Ethanol in Gegenwart von sauren Katalysatoren.

Aus der DE-OS 19 10 473 ist es bekannt, tert.Butanol in der Weise herzustellen, daß das Isobutylen bei mäßig erhöhter Temperatur durch Umsetzung mit höchstens 50 %iger wäßriger Schwefelsaure hydratisiert wird und das gebildete tert.-Butanol anschließend durch Vakuumdestillation als Azeotrop mit 12 % Wasser aus der erhaltenen wäßrig-schwefelsauren tert.-Butanollösung abgetrennt wird. Dieses Verfahren hat den Nachteil, daß danach nur ein wasserhaltiges tert.-Butanol erhalten wird. Zur Vermeidung der Rückspaltung des tert.-Butanols in der schwefelsauren Lösung ist die Anwendung eines guten Vakuums bei der destillativen Abtrennung des tert.-Butanols aus der wäßrig--schwefelsauren tert.-Butanollösung erforderlich, wodurch das Verfahren relativ aufwendig wird. Außerdem hat das Verfahren den Nachteil der hohen Korrosionsneigung wäßriger Schwefelsäure, weswegen Blei oder andere korrosionsfeste Materialien verwendet werden müssen.

Es ist weiter bekannt, z.B. aus der DE-OS 30 24 147, daß Spuren von Wasser bei der Herstellung von Methyltert.-butylether aus Isobuten und Methanol zu tert.-Butanol umgesetzt werden. Die tert.-Butanol-Spuren müssen gemeinsam mit Methanol extrahiert und der Extrakt destillativ getrennt werden. Dies erfordert neben hohem Energieaufwand hohen apparativen Aufwand zur Reinigung des Waschwassers. Außerdem werden nur geringe Mengen an tert.-Butanol gewonnen.

Es wurde nun ein vorteilhaftes Verfahren gefunden zur Herstellung von tert.-Butanol durch Umsetzung von Isobuten enthaltenden $C_4$-Kohlenwasserstoffgemischen mit Wasser in Gegenwart von sauren Ionenaustauschern und Abtrennung des gebildeten tert.-Butanols aus dem Reaktionsgemisch, welches dadurch gekennzeichnet ist, daß man einen Teil des bei der Hydratisierung gebildeten tert.-Butanols in die Hydratisierung zurückführt.

Nach dem neuen Verfahren wird ein praktisch wasserfreies tert.-Butanol in sehr guter Ausbeute und von hoher Reinheit gewonnen.

In einer vorteilhaften Ausführungsform des Verfahrens wird die Umsetzung des Isobuten enthaltenden $C_4$-Kohlenwasserstoffgemisches gemeinsam mit Wasser und Methanol und/oder Ethanol unter Bildung von tert.-Butanol und Methyl-tert.-butylether und/oder Ethyl-tert.-butylether durchgeführt. Zweckmäßig wird das nicht umgesetzte $C_4$-Kohlenwasserstoffgemisch von dem Reaktionsgemisch destillativ abgetrennt, das in dem bei der Destillation erhaltenen $C_4$-Kohlenwasserstoffgemisch enthaltene Methanol und/oder Ethanol durch eine Wasserwäsche abgetrennt und das bei der Wasserwäsche erhaltene Waschwasser als Reaktand für die Hydratisierungsumsetzung mit dem Isobuten enthaltenden $C_4$-Kohlenwasserstoffgemisch eingesetzt. Dabei kann das erhaltene Methanol und/oder Ethanol enthaltende Waschwasser vor dem Einsatz für die Hydratisierungsumsetzung zweckmäßig durch Destillation in eine Alkoholfraktion und eine Wasserfraktion aufgetrennt werden und nur die Wasserfraktion für die Hydratisierungsumsetzung eingesetzt werden. Vorteilhaft wird jedoch das aus der Wasserwäsche erhaltene Waschwasser ohne vorherige Abtrennung des darin enthaltenen Methanols und/oder Ethanols für die Umsetzung mit dem Isobuten enthaltenden $C_4$-Kohlenwasserstoffgemisch eingesetzt.

Es ist ein besonderer Vorteil des erfindungsgemäßen Verfahrens, daß die bei den bekannten Verfahren zur Methyl-tert.-butylether-Herstellung erforderliche aufwendige Aufarbeitung des aus der Wasserwäsche erhaltenen methanolhaltigen Waschwassers beim vorliegenden Verfahren entfallen kann, wenn die Methyl-tert.-butylether-Herstellung mit der gleichzeitigen Herstellung von tert.-Butanol verbunden wird. Es ist ein weiterer Vorteil des erfindungsgemäßen Verfahrens, daß die gemeinsame Herstellung von tert.-Butanol und Methyl-tert.-butylether (MTBE) und/oder Ethyl-tert.-butylether nicht die bei einer kuppelproduktion üblicherweise auftretenden Probleme der mengenmäßigen Abstimmung der beiden kuppelprodukte macht, da tert.-Butanol und MTBE beide für den gleichen Zweck verwendet werden können, nämlich als oktanzahlsteigernder Benzinzusatz. Der Nachteil des tert.-Butanols bei dieser Verwendung, der hohe Schmelzpunkt, kann durch Abmischung mit MTBE vermindert oder gar aufgehoben werden. Andererseits verbessern sich auch die lösungsvermittelnden Eigenschaften des MTBE durch eine Abmischung mit tert.-Butanol.

Für das erfindungsgemäße Verfahren geeignete Isobuten enthaltende $C_4$-Kohlenwasserstoffgemische werden beispielsweise beim thermischen oder katalytischen Cracken von Erdölprodukten, bei der Herstellung von Ethylen durch Pyrolyse von verflüssigtem Petroleumgas (LPG), Leichtbenzin (Naphtha), Gasöl oder dgl. oder bei der katalytischen Dehydrierung von n-Butan und/oder n-Buten erhalten. Diese $C_4$-Kohlenwasserstoffgemische enthalten in der Regel neben dem Isobuten olefinische und paraffinische $C_4$-Kohlenwasserstoffe und können darüber hinaus noch Butadien, z.B. in einer Menge von bis zu 70 Gew. %. und höhere Acetylene, wie Butin-1 und Butenin enthalten. Butadien enthaltende $C_4$-Kohlenwasserstoffgemische können als solche oder nach vorheriger Abtrennung des Butadiens aus dem $C_4$-Kohlenwasserstoffgemisch, z.B. durch Butadien-Extraktion unter Verwendung eines selektiven Lösungsmittels, verwendet werden. Die $C_4$-Kohlenwasserstoffgemische können außerdem noch $C_3$-Kohlenwasserstoffe wie Propan, Propen, Propin z.B. bis zu 10 Gew.%, enthalten. Die $C_4$-Kohlenwasserstoffgemische weisen im allgemeinen einen Isobutengehalt von 5 bis 95 Gew.%, vorzugsweise 10 bis 90 Gew.%, insbesondere 20 bis 70 Gew.%, auf. Vorzugsweise werden solche $C_4$-Kohlenwasserstoffgemische verwendet, die neben Isobuten n-Butan, Isobutan, Buten-1, trans-Buten-2 und cis-Buten-2 und gegebenenfalls Butadien-1,3 enthalten. Nach dem erfindungsgemäßen Verfahren wird das Isobuten in ausgezeichneter Ausbeute außerordentlich selektiv hydratisiert. In den $C_4$-Kohlenwasserstoffgemischen enthaltene Butene und enthaltenes Butadien-1,3 reagieren dabei nur in Spuren unter Bildung von sekundär-Butanol und Buten-1-ol-3.

Als saure Ionenaustauscher für die Hydratisierung und gegebenenfalls Veretherung des Isobutens nach dem erfindungsgemäßen Verfahren kommen beispielsweise sulfonierte Kohlen, sulfonierte Phenol-Formaldehyd-Harze, sulfonierte von Cumaron-Inden-Kondensationsprodukten abgeleitete Harze sowie insbesondere sulfonierte Polystyrol-Harze wie Kern-sulfonierte vernetzte Styrol-Divinylbenzol-Copolymerisate in Betracht. Die Ionenaustauscher werden zweckmäßig in der Wasserstoff-Form verwendet. Die Menge der sauren Ionenaustauscher beträgt im allgemeinen 0,01 bis 1 l Schüttvolumen pro l Reaktorvolumen. Die sauren Ionenaustauscher werden im allgemeinen als solche eingesetzt. Sie können jedoch auch auf einem Trägermaterial verwendet werden. Geeignete Trägermaterialien sind z.B. Aluminiumoxid, Kieselsäure, Aktivkohle. Für die Hydratisierung und gegebenenfalls Veretherung können z.B. Rührkessel-oder Festbettreaktoren verwendet werden. Vorzugsweise werden Festbettreaktoren eingesetzt.

Die erfindungsgemäße Hydratisierung und gegebenenfalls Veretherung mit Methanol und/oder Ethanol wird im allgemeinen bei Temperaturen von 20 bis 120°C, vorzugsweise 30 bis 110°C, insbesondere 40 bis 80°C, durchgeführt.

Die Hydratisierung und gegebenenfalls Veretherung des Isobutens kann bei Normaldruck erfolgen. Es ist jedoch zweckmäßig, einen geringen Überdruck, z.B. von 1,01 bis 30 bar, vorzugsweise 2 bis 20 bar, insbesondere 2 bis 10 bar, anzuwenden. Das Isobuten enthaltende $C_4$-Kohlenwasserstoffgemisch kann dabei je nach Druck und Temperatur für die Umsetzung flüssig oder gasförmig mit dem Ionenaustauscher in Berührung gebracht werden. Vorzugsweise werden flüssige Isobuten enthaltende $C_4$-Kohlenwasserstoffgemische mit dem sauren Ionenaustauscher in Berührung gebracht. Die reziproke Verweilzeit, bezogen auf den leeren Reaktor (LHSV), beträgt im allgemeinen 0,1 bis 10 $h^{-1}$, vorzugsweise 0,8 bis 3 $h^{-1}$.

Die zur Hydratisierung eingesetzte Menge Wasser beträgt im allgemeinen 0,1 bis 5 Mol, vorzugsweise 0,5 bis 3 Mol, insbesondere 0,8 bis 2 Mol der stöchiometrischen Menge. Falls das Isobuten gemeinsam hydratisiert und mit Methanol und/oder Ethanol verethert wird, beträgt das Molverhältnis von Wasser zu Methanol und/oder Ethanol zweckmäßig 100:1 bis 1:10, vorzugsweise 50:1 bis 1:5, insbesondere 10:1 bis 1:1.

Bei dem erfindungsgemäßen Verfahren wird ein Teil des bei der Hydratisierung gebildeten tert.-Butanols in die Hydratisierung zurückgeführt. Im allgemeinen wird eine solche Menge tert.-Butanol in die Hydratisierung zurückgeführt, daß das Gewichtsverhältnis des zurückgeführten tert.-Butanols zu dem für die Hydratisierung eingesetzen Isobuten enthaltenden $C_4$-Kohlenwasserstoffgemisch 1:10 bis 50:1, vorzugsweise 1:5 bis 1:30, insbesondere 1:2 bis 1:30, beträgt.

Das bei der Hydratisierunng und gegebenenfalls Veretherung erhaltene Reaktionsgemisch wird zweckmäßig durch Destillation aufgetrennt. Dabei wird das gebildete tert.-Butanol zweckmäßig als Sumpfprodukt oder tiefliegeder Seitenabzug, zweckmäßig im unteren Drittel der Destillationszone abgezogen. Das tert.-Butanol kann als reines Produkt abgezogen werden. Es kann jedoch auch bei gemeinsamer Hydratisierung und Veretherung Methyl-und/oder Ethyl-tert.-butylether und gegebenenfalls nicht umgesetztes Methanol und/oder Ethanol enthalten, falls dies erwünscht ist. In einer vorteilhaften Ausführungsform des Verfahrens wird die Wassermenge für die Hydratisierung so bemessen, daß die Menge des nicht umgesetzten Wassers im aus der Hydratisierung erhaltenen Reaktionsgemisch nicht höher als die Wassermenge ist, die der Wassermenge im sich bei der Destillation bildenden Wasser/$C_4$-Kohlenwasserstoff-Azeotrop entspricht. Bei dieser Arbeitsweise läßt sich das Wasser in der Destillationskolonne über Kopf als Wasser/$C_4$-Kohlenwasserstoff-Azeotrop abtrennen. Hierdurch kann die Bildung eines Wasser/tert.-Butanol-Azeotrops vermieden werden, wodurch eine aufwendige Wasserabtrennung, z.B. durch Extraktion, die bei der Bildung eines Wasser/tert.-Butanol-Azeotrops erforderlich gewesen wäre, um aus dem wasserhaltigen tert.-Butanol wasserfreies tert.-Butanol zu erhalten, entfällt. Das am Kopf der Destillation erhaltene Produkt enthält im allgemeinen die nicht umgesetzten Kohlenwasserstoffe, gegebenenfalls Methanol und/oder Ethanol und gegebenenfalls Methyl-und/oder Ethyl-tert.-butylether. Das Kopfprodukt der Destillation wird zweckmäßig kondensiert, wobei sich darin enthaltenes Wasser in der Regel als gegebenenfalls Methanol und/oder Ethanol enthaltende wäßrige Phase abscheidet. Die wäßrige Phase wird zweckmäßig zur Hydratisierung zurückgeführt.

Es kann vorteilhaft sein, daß das bei der Destillation des bei der Hydratisierung und Veretherung erhaltenen Reaktionsgemischs die nicht umgesetzten Kohlenwasserstoffe und Methanol und/oder Ethanol enthaltende Kopfprodukt zur Abtrennung des Methanols und/oder Ethanols einer Wasserwäsche unterzogen wird. Das bei der Wasserwäsche erhaltene Waschwasser wird zweckmäßig als Reaktand für die Hydratisierungsumsetzung mit dem Isobuten enthaltenden Einsatz-$C_4$-Kohlenwasserstoffgemisch eingesetzt. Dabei wird das Waschwasser zweckmäßig ohne vorherige Abtrennung des im Waschwasser enthaltenen Methanols und/oder Ethanols für die Umsetzung mit dem $C_4$-Kohlenwasserstoffgemisch eingesetzt.

In einer vorteilhaften Ausführungsform des Verfahrens wird das vom Reaktionsgemisch der Hydratisierung und gegebenenfalls Veretherung abgetrennte, nicht umgesetzte $C_4$-Kohlenwasserstoffgemisch zur weiteren Umsetzung von noch nicht umgesetztem Isobuten in einer zweiten Umsetzungsstufe mit Methanol und/oder Ethanol unter Bildung des Methyl-und/oder Ethyl-tert.-butylethers umsetzt.

Für die in der zweiten Umsetzungsstufe durchgeführte Veretherung werden zweckmäßig die Reaktionsbedingungen, die vorstehend für die Veretherung in der ersten Umsetzungsstufe beschrieben worden sind, z.B. bezüglich der Katalysatoren, Drücke und Temperaturen, entsprechend angewendet. Von dem in der zweiten Umsetzungsstufe erhaltenen Reaktionsgemisch wird das nicht umgesetzte $C_4$-Kohlenwasserstoffgemisch zweckmäßig destillativ abgetrennt, das in dem abgetrennten $C_4$-Kohlenwasserstoffgemisch enthaltene Methanol und/oder Ethanol durch eine Wasserwäsche abgetrennt und das bei der Wasserwäsche erhaltene Waschwasser als Reaktand für die Hydratisierungsumsetzung in der ersten Umsetzungsstufe mit dem Isobuten enthaltenden $C_4$-Kohlenwassergemisch eingesetzt. Vorteilhaft wird dabei das Waschwasser ohne vorherige Abtrennung des im Waschwasser enthaltenen Methanols und/oder Ethanols eingesetzt.

Durch die Veretherung in der zweiten Umsetzungsstufe wird ein die nicht umgesetzten Kohlenwasserstoffe enthaltendes $C_4$-Kohlenwasserstoffgemisch, das praktisch Isobuten-frei ist, erhalten. Solche $C_4$-Kohlenwasserstoffegemische können mit Vorteil beispielsweise für die Gewinnung von 1-Buten eingesetzt werden.

Die erfindungsgemäß auszuführenden Wasserwäschen zur Abtrennung von Methanol und/oder Ethanol aus den bei der Destillation der bei der Veretherung erhaltenen Reaktionsgemische erhaltenen $C_4$-Kohlenwasserstoffgemischen erfolgt in üblicher Weise durch Behandlung des Methanol und/oder Ethanol enthaltenden $C_4$-Kohlenwasserstoffgemischs mit Wasser, z.B. in einem Rührkessel oder vorzugsweise durch Gegenstrom-Extraktion.

Eine Ausführungsform des erfindungsgemäßen Verfahrens besteht beispielsweise darin, daß ein isobutenhaltiges $C_4$-Kohlenwasserstoffgemisch mit Wasser in der Hydratisierungsreaktion an einem sauren Ionenaustauscher unter Bildung von tert.-Butanol umgesetzt wird. Das Reaktionsprodukt wird destilliert und das dabei erhaltene tert.-Butanol teilweise zur Hydratisierungsstufe zurückgeführt ebenso wie die sich bei der Kondensation der als Kopfprodukt der Destillation erhaltenen $C_4$-Kohlenwasserstoff-Fraktion abscheidende wäßrige Phase. Die nicht umgesetztes Isobuten enthaltente $C_4$-Kohlenwasserstoff-Fraktion wird anschließend in einer zweiten Stufe an einem sauren Ionenaustauscher umgesetzt, und das erhaltene Reaktionsprodukt wird destilliert. Die als Kopfprodukt erhaltene, die nicht umgesetzten Kohlenwasserstoffe enthaltende Fraktion wird zur Entfernung von darin enthaltenem Methanol und/oder Ethanol mit Wasser gewaschen, und das Methanol und/oder Ethanol enthaltende Waschwasser wird in die Hydratisierungsstufe zurückgeführt.

Das folgende Beispiel veranschaulicht die Erfindung.

Beispiel

Durch einen vollständig mit sulfoniertem Polystyroldivinylbenzol-Harz in der Wasserstofform (Lewatit SPC 118, Kornfraktion 0,8 bis 1 mm) gefüllten Festbettrohrreaktor von 450 cm³ Volumen wurden bei 50°C 108 g/h tert.-Butanol mit 10 Gew.% Wasser und 10 Gew.% Methanol, jeweils bezogen auf das tert.-Butanol, sowie 72 g/h eines C₄-Kohlenwasserstoffgemisches mit folgender Zusammensetzung geleitet:

2,9 Gew.% Isobutan, 25,6 Gew.% 1-Buten, 10,3 Gew.% n-Butan, 45,2 Gew.% Isobuten, 9,2 Gew.% 2-Buten-trans, 6,7 Gew.% 2-Buten-cis, 0,1 Gew.% 1,3-Butadien.

Das aus der Hydratisierungs-/Veretherungsstufe erhaltene Reaktionsprodukt wurde destilliert, wobei wasserfreies tert.-Butanol am Boden der Destillation erhalten wurde. Von dem erhaltenen tert.-Butanol wurde ein Teil in die Hydratisierungs-/Veretherungsstufe zurückgeführt. Am Kopf der Destillation wurde eine die nicht umgesetzten C₄-Kohlenwasserstoffe enthaltende Fraktion abgezogen, aus der sich nach der Kondensation eine methanolhaltige wäßrige Phase abschied, die ebenfalls in die Hydratisierungs-/Veretherungsstufe zurückgeführt wurde.

Die die nicht umgesetzten C₄-Kohlenwasserstoffe enthaltende Fraktion enthielt neben Methanol und Methyl-tert.-butylether noch 13,3 Gew.% Isobuten. Diese Fraktion wurde anschließend in einer weiteren Reaktionsstufe durch einen zweiten Festbettreaktor, in dem eine Temperatur von 70°C aufrechterhalten wurde und der mit dem gleichen Katalysator wie der erste Reaktor gefüllt war, zusammen mit der für die Veretherung stöchiometrischen Methanolmenge geleitet. Das aus der weiteren Reaktionsstufe erhaltene Reaktionsprodukt wurde ebenfalls destilliert, wobei am Sumpf der Methyl-tert.-butylether und am Kopf ein die nicht umgesetzten C₄-Kohlenwasserstoffe enthaltendes Gemisch erhalten wurden. Das erhaltene C₄-Kohlenwasserstoffgemisch wurde zur Abtrennung von darin enthaltenem Methanol mit Wasser gewaschen. Das erhaltene methanolhaltige Wasser wurde in die erste Reaktionsstufe zurückgeführt, in der das zurückgeführte Waschwasser zusammen mit dem aus der Destillation des Reaktionsproduktes der ersten Reaktionsstufe erhaltenen Wasser als Reaktand für die Hydratisierung eingesetzt wurde, so daß ein stationärer Betrieb aufrechterhalten werden konnte.

**Ansprüche**

1. Verfahren zur Herstellung von tert.-Butanol durch Umsetzung von Isobuten enthaltenden C₄-Kohlenwasserstoffgemischen mit Wasser in Gegenwart von sauren Ionenaustauschern und Abtrennung des gebildeten tert.-Butanols aus dem Reaktionsgemisch, **dadurch gekennzeichnet, daß** man einen Teil des bei der Hydratisierung gebildeten tert.-Butanols in die Hydratisierung zurückführt.

2. Verfahren nach Anspruch 1, **dadurch gekennzeichent, daß** die Umsetzung des Isobuten enthaltenden C₄-Kohlenwasserstoffgemischs gemeinsam mit Wasser und Methanol und/oder Ethanol unter Bildung von tert.-Butanol und Methyl-tert.-butylether und/oder Ethyl-tert.-butylether durchgeführt wird.

3. Verfahren nach Anspruch 2, **dadurch gekennzeichnet, daß** man das nicht umgesetzte C₄-Kohlenwasserstoffgemisch von dem Reaktionsgemisch destillativ abtrennt, das in dem bei der Destillation erhaltenen C₄-Kohlenwasserstoffgemisch enthaltene Methanol und/oder Ethanol durch eine Wasserwäsche abtrennt und das bei der Wasserwäsche erhaltene Waschwasser als Reaktand für die Hydratisierungsumsetzung mit dem Isobuten enthaltenden C₄-Kohlenwasserstoffgemisch einsetzt.

4. Verfahren nach Anspruch 3, **dadurch gekennzeichnet, daß** das Waschwasser ohne vorherige Abtrennung des im aus der Wasserwäsche erhaltenen Waschwasser enthaltenen Methanols und/oder Ethanols für die Umsetzung mit dem Isobuten enthaltenden C₄-Kohlenwasserstoffgemisch eingesetzt wird.

5. Verfahren nach Anspruch 1 bis 4, **dadurch gekennzeichnet, daß** man das vom Reaktionsgemisch abgetrennte nicht umgesetzte C₄-Kohlenwasserstoffgemisch zur weiteren Umsetzung von noch nicht umgesetztem Isobuten in einer zweiten Umsetzungsstufe mit Methanol und/oder Ethanol umsetzt.

6. Verfahren nach Anspruch 5, **dadurch gekennzeichnet, daß** man von dem in der zweiten Umsetzungsstufe erhaltenen Reaktionsgemisch das nicht umgesetzte C₄-Kohlenwasserstoffgemisch destillativ abtrennt, das in dem abgetrennten C₄-Kohlenwasserstoffgemisch enthaltene Methanol und/oder Ethanol durch eine Wasserwäsche abtrennt und das bei der Wasserwäsche erhaltene Waschwasser als Reaktand für die Hydratisierungsumsetzung mit dem Isobuten enthaltenden C₄-Kohlenwasserstoffgemisch einsetzt.

7. Verfahren nach Anspruch 6, **dadurch gekennzeichnet, daß** das Wasch7. wasser ohne vorherige Abtrennung des im Waschwasser nach der Wasserwäsche erhaltenen Methanols und/oder Ethanols für die Umsetzung mit dem Isobuten enthaltenden C₄-Kohlenwasserstoffgemische eingesetzt wird.